# EUROPEAN PATENT APPLICATION

(11) **EP 1 953 141 A1**
(43) Date of publication of application: **06.08.2008**
(21) Application number: 07384015.9
(22) Date of filing: 31.01.2007
(51) Int. Cl.: C07D 209/16, A61K 31/404, A61P 3/04, A61P 25/28

(54) **Aryl-substituted sulfonamides for the treatment of cognitive or food ingestion related disorders**

(71) Applicant: Laboratorios del Dr. Esteve S.A., 08041 Barcelona (ES)
(72) Inventor: Diaz-Fernandez, José Luis, 08240 Manresa (Barcelona) (ES)
(74) Representative: Peters, Hajo

(57) **Abstract**

The present invention relates to aryl substituted sulfonamides with 5-HT₆ receptor affinity, a medicament comprising a said compound, and the use of one of said compounds for the manufacture of a medicament.

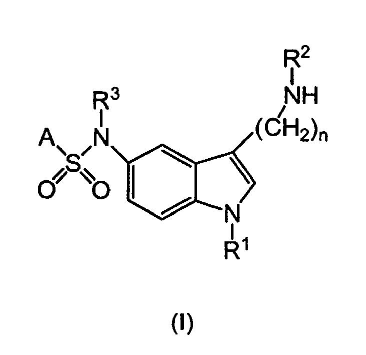

## Description

The present invention relates to aryl substituted sulfonamides with 5-HT₆ receptor affinity, a medicament comprising a said compound, and the use of one of said compounds for the manufacture of a medicament.

The superfamily of serotonin receptors (5-HT) includes 7 classes (5-HT₁-5-HT₇) encompassing 14 human subclasses [D. Hoyer, et al., Neuropharmacology, 1997,36,419]. The 5-HT₆ receptor is a serotonin receptor identified by molecular cloning both in rats [F.J. Monsma, et al., Mol. Pharmaco/., 1993, 43, 320; M. Ruat, et al., Biochem. Biophys. Res. Commun., 1993, 193, 268] and in humans [R. Kohen, et al., J. Neurochem., 1996, 66,47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NYAcad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther. , 1995, 274, 173; A.J. Sleight, et al., Behav. Brain Res. , 1996, 73, 245; T.A. Branchek, et al., nnu. Rev. Pharmacol. Toxicol. , 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J. Mosma, et al., Mol. Pharmacol. , 1993, 43, 320; T. Shinkai, et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol. , 2000, 130, 1597; C. Gérard, et al., Brain Research , 1997, 746, 207; M.R. Pranzatelli, Drugs of Today , 1997, 33, 379]. Moreover, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases.

Thus, the object of the present invention was to provide medicaments that comprise compounds with 5-HT₆ receptor affinity and which are suitable for the prophylaxis and/or treatment of food-ingestion related disorders.

In particular, it was an object of the present invention to provide a medicament suitable for the prophylaxis and/or treatment of obesity/food ingestion disorders, which preferably does not show the undesired side effects of the conventional medicaments, or at least less frequent and/or less pronounced.

It has been found that the compounds of general formula (I) given below show affinity for the 5-HT₆-receptor. These compounds are therefore also suitable for the manufacture of a medicament for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity or for the manufacture of a medicament for the prophylaxis and/or treatment of cognitive disorders.

Compounds showing similarities to the compounds according to the invention are being found in EP 1 445 252 A1, describing also compounds binding to the 5HT₆-receptor.

Said object has been achieved by providing as an active substance an aryl-substituted sulfonamide compound according to general formula (I) 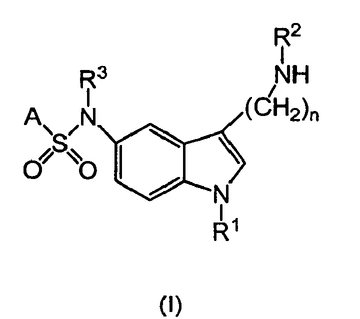 wherein
A represents an optionally at least mono substituted aryl radical, which may be bonded via an optionally at least mono-substituted or unsaturated alkylene group; an optionally at least monosubstituted alkyl-diaryl radical or an optionally at least monosubstituted group 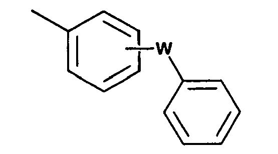 , wherein **W** represents a bond between the two rings, CH₂, O, S and NH;
**R¹** represents hydrogen, or C₁-C₄ alkyl or a benzyl radical;
**R²** represents hydrogen, or C₁-C₄ alkyl;
**R³** represents hydrogen or C₁-C₄ alkyl;
**n** represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

It is particularly preferred if the following proviso applies:
- if R¹, R² and R³ are all H, A may only be alkyl- or halogen-substituted naphthyl or unsubstituted β-naphthyl, an optionally at least monosubstituted alkyl-diaryl radical or an optionally at least monosubstituted group

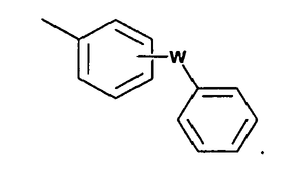

An "aryl", "aryl radical" or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

An "alkyl-diaryl radical" is understood as meaning a structure in which two aryl radicals are bonded to one C-atom of an alkyl-chain or alkylene and are bonded to the core structure through this alkyl-chain or alkylene. Possible examples include:

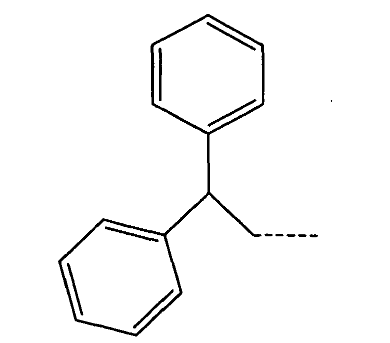

In connection with aryls, aryl groups or aryl radicals, "substituted" is understood - unless defined otherwise - as meaning replacement of at least one hydrogen radical on the aryl radical by OH, SH, =O, halogen (F, CI, Br, I), CN, NO₂, COOH; NRₓR_{y}, with Rₓ and Ry independently being either H or a saturated or unsaturated, linear or branched, substituted or unsubstituted C₁₋₆-alkyl; by a saturated or unsaturated, linear or branched, substituted or unsubstituted C₁₋₆-alkyl; a saturated or unsaturated, linear or branched, substituted or unsubstituted -O-C₁₋₆₋alkyl (alkoxy); a saturated or unsaturated, linear or branched, substituted or unsubstituted -S-C₁₋₆₋alkyl; a saturated or unsaturated, linear or branched, substituted or unsubstituted -C(O)-C₁₋₆₋alkyl; a saturated or unsaturated, linear or branched, substituted or unsubstituted -C(O)-O-C₁₋₆₋alkyl; a substituted or unsubstituted phenyl. Within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents. Therefore, "optionally at least monsubstituted" means either "not substituted" if the option is not fulfilled, "monosubstituted" or "polysubstituted".

In the context of this invention "alkyl", "alkyl radical" or group is understood as meaning saturated and unsaturated, linear or branched hydrocarbons, which can be unsubstituted or mono- or polysubstituted. Thus unsaturated alkyl is understood to encompass alkenyl and alkinyl groups, like e.g. -CH=CH-CH₃ or -C=C-CH₃, while saturated alkyl encompasses e.g. - CH₃ and -CH₂-CH₃. In these radicals, C₁₋₂-alkyl represents C₁- or C₂-alkyl, C₁₋₃-alkyl represents C₁-, C₂- or C₃-alkyl, C₁₋₄-alkyl (C₁-C₄-alkyl) represents C₁-, C₂-, C₃- or C₄-alkyl, C₁-₅-alkyl represents C₁-, C₂-, C₃-, C₄-, or C₅-alkyl, C₁₋₆-alkyl represents C₁-, C₂-, C₃-, C₄-, C₅- or C₆-alkyl, C₁₋₇-alkyl represents C₁-, C₂-, C₃-, C₄-, C₅-, C₆- or C₇-alkyl, C₁₋₈-alkyl represents C₁-, C₂-, C₃-, C₄-, C₅-, C₆-, C₇- or C₈-alkyl, C₁₋₁₀-alkyl represents C₁-, C₂-, C₃-, C₄-, C₅-, C₆-, C₇-, C₈-, C₉- or C₁₀-alkyl and C₁₋₁₈-alkyl represents C₁-, C₂-, C₃-, C₄-, C₅-, C₆-, C₇-, C₈-, C₉-, C₁₀-, C₁₁-, C₁₂-, C₁₃-, C₁₄-, C₁₅-, C₁₆-, C₁₇- or C₁₈-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

The term "alkylene" is understood as meaning a divalent alkyl group like -CH₂- or -CH₂-CH₂-. with (CH₂)₃₋₆ being understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning - CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc. "Alkylene" might also include a non-saturated divalent alkyl-chain.

In connection with "alkylene", "alkyl", "alkyl radical" or group - unless defined otherwise - the term "substituted" in the context of this invention is understood as meaning replacement of at least one hydrogen radical by F, Cl, Br, 1, NH₂, SH or OH; within that "monosubstituted" means the substitution of exactly one hydrogen radical, whereas "polysubstituted" means the substitution of more than one hydrogen radical with "polysubstituted" radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. Therefore, "optionally at least monsubstituted" means either "not substituted" if the option is not fulfilled, "monosubstituted" or "polysubstituted".

The term "salt' is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

Particularly preferred are aryl-substituted sulfonamide compound according to the invention according to formula I, wherein
A represents an optionally at least monosubstituted naphthyl or
a group selected from among: 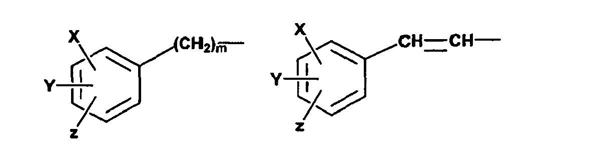 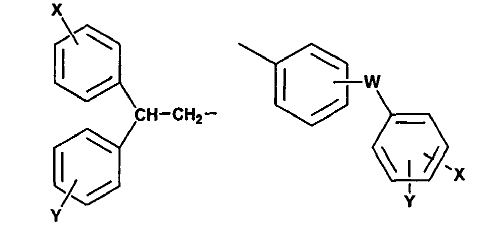 with
**X**, **Y** and **Z** independently representing hydrogen, fluorine, chlorine, bromine, Iodine, a C₁-C₄ alkyl, a C₁-C₄,alkoxy, a C₁-C₄ alkylthio, trifluoromethyl, cyano, nitro, OH, SH and NH₂;
**W** representing a bond between the two rings, CH₂, O, S and NH;
**m** represents 0, 1, 2, 3 or 4.

Further very preferred is an aryl-substituted sulfonamide compound according to the invention according to formula I 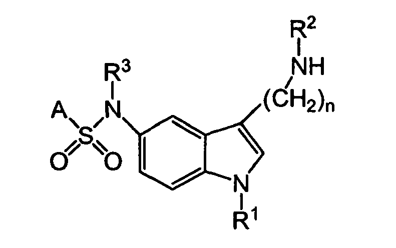 , wherein
**A** represents an optionally at least mono-substituted naphthyl;
**R¹** represents hydrogen, or C₁-C₄ alkyl;
**R²** represents hydrogen, or C₁-C₄ alkyl;
**R³** represents hydrogen or C₁-C₄ alkyl;
**n** represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
with the proviso that
if R¹, R² and R³ are all H, A may only be alkyl- or halogen-substituted naphthyl or unsubstituted β-naphthyl.

In this regard it is also very preferred if this aryl-substituted sulfonamide compound according to the invention according to formula I is a compound, wherein
**A** represents
   a naphthyl, unsubstituted or substituted by 1 or 2 radicals selected from halogen, C₁-C₄ alkyl, O-C₁-C₄ alkyl, OCF₃, CF₃, CHF₂, CN, NO₂, OH, SH, NH₂, phenyl, optionally at least monosubstituted by halogen, C₁-C₄ alkyl, O-C₁-C₄ alkyl, OH, SH, NH₂, or a monocyclic heterocyclic ring system with 5 or 6 ring members containing 1 or 2 atoms of oxygen, nitrogen or sulphur as ring member, at least monosubstituted by halogen, C₁-C₄ alkyl, O-C₁-C₄ alkyl, OH, SH, NH₂;
      preferably
   a naphthyl, unsubstituted or substituted by 1 or 2 radicals selected from halogen, C₁-C₄ alkyl, CF₃, CHF₂, O-C₁-C₄ alkyl, OCF₃, OH, SH, or NH₂.

Further very preferred is an aryl-substituted sulfonamide compound according to the invention according to formula I, wherein
**R¹** represents hydrogen, CH₃, C₂H₅, C₃H₇ or C₄H₉; preferably hydrogen, methyl, ethyl, n-propyl, i-propyl, n-butyl, i-butyl and t-butyl; more preferably hydrogen, methyl or ethyl; most preferably hydrogen or methyl.

Further very preferred is an aryl-substituted sulfonamide compound according to the invention according to formula I, wherein
**R²** represents hydrogen, CH₃, C₂H₅, C₃H₇ or C₄H₉; preferably hydrogen, methyl, ethyl, n-propyl, i-propyl, n-butyl, i-butyl and t-butyl; more preferably hydrogen, methyl or ethyl.

Further very preferred is an aryl-substituted sulfonamide compound according to the invention according to formula I, wherein
**R³** represents hydrogen, CH₃, C₂H₅, C₃H₇ or C₄H₉; preferably hydrogen, methyl, ethyl, n-propyl, i-propyl, n-butyl, i-butyl and t-butyl; more preferably hydrogen, methyl or ethyl; most preferably hydrogen.

Further very preferred is an aryl-substituted sulfonamide compound according to the invention according to formula I, wherein
**n** represents 0, 1 or 2, preferably 2.

A further particularly preferred embodiment of the invention is an aryl-substituted sulfonamide compound according to general formula II, 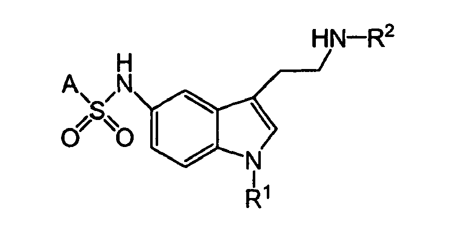 wherein
**A** represents
   a naphthyl, unsubstituted or substituted by 1 or 2 radicals selected from halogen, C₁-C₄ alkyl, CF₃, CHF₂, O-C₁-C₄ alkyl, OCF₃, OH, SH, or NH₂; and
**R¹** represents hydrogen or CH₃;
**R²** represents hydrogen, CH₃, C₂H₅, C₃H₇ or C₄H₉;
optionally in form of a salt, preferably a physiologically acceptable salt thereof, or a corresponding solvate, respectively;

It is highly preferred if for the aryl-substituted sulfonamide compound according to general formula II the following proviso applies:
if R¹, R² and R³ are all H, A may only be alkyl- or halogen-substituted naphthyl or unsubstituted β-naphthyl.

Further very preferred is an aryl-substituted sulfonamide compound according to the invention according to formula II, wherein
**R²** represents methyl, ethyl, n-propyl, i-propyl, n-butyl, i-butyl and t-butyl; preferably methyl or ethyl.
   or
**R²** represents hydrogen.

Further very preferred is an aryl-substituted sulfonamide compound according to the invention according to formula II, wherein the compound is selected from
Naphthalene-1-sulfonic acid [3-(2-ethylamino-ethyl)-1H-indol-5-yl]-amide
Naphthalene-1-sulfonic acid [3-(2-methylamino-ethyl)-1H-indol-5-yl]-amide
5-Chloro-naphthalene-2-sulfonic acid [3-(2-ethylamino-ethyl)-1H-indol-5-yl]-amide
5-Chloro-naphthalene-2-sulfonic acid [3-(2-methylamino-ethyl)-1H-indol-5-yl]-amide
5-Chloro-naphthalene-2-sulfonic acid [1-methyl-3-(2-methylamino-ethyl)-1H-indol-5-yl]-amide
or from
Naphthalene-2-sulfonic acid [3-(2-amino-ethyl)-1H-indol-5-yl]-amide
5-Chloro-naphthalene-2-sulfonic acid [3-(2-amino-ethyl)-1 H-indol-5-yl]-amide
5-Chloro-naphthalene-2-sulfonic acid [3-(2-amino-ethyl)-1-methyl-1H-indol-5-yl]-amide;
optionally in form of a salt, preferably a physiologically acceptable salt thereof, or a corresponding solvate, respectively.

A further aspect of this invention provides a production process for the compounds according to the invention as exemplified in the experimental part of the examples (see below).

The compounds general formula (I) according to the invention, wherein R¹, R², R³, n and A are as indicated above, can be prepared by reacting a compound with the general formula (V) or one of its suitably protected derivatives 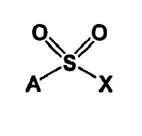 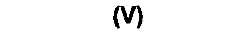 wherein A is as indicated above for the general formula (I) and X is an acceptable salient group including a halogen atom, particularly chlorine;
with a 5-aminoindol with the general formula (VI), or one of its suitably protected derivatives; 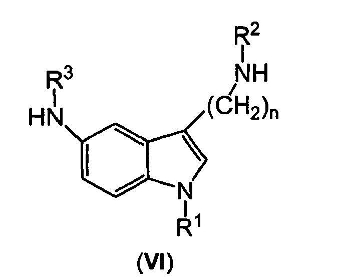 wherein n, R₁, R₂ and R₃ are as indicated above for the general formula (I);
in order to obtain the corresponding sulfonamide and, optionally, eliminating from it the protective groups and / or forming a pharmacologically acceptable salt.

It is preferred that if the compounds according to formulas (V) or (VI) carry a chemical substituent needing protection during the following reaction the substituent is being protected by a protective group, especially those which are well-known to the expert and in the state of the art. Particularly preferred if the hydrogen on the NH-group to which is bound R² in the compound according to formula (VI) is protected by a protective group, e.g. a Boc-group.

The reaction between the compounds with the general formula (V) and (VI) is carried out in the presence of an organic solvent such as an alkyl ether, particularly diethyl ether, or a cycloalkyl, particularly tetrahydrofurane or dioxane, a halogenated organic hydrocarbon, particularly methylene chloride or chloroform, an alcohol, particularly methanol or ethanol, an aprotic dipolar solvent, particularly acetonitrile, pyridine or dimethylformamide, or any other suitable solvent.

The reaction preferably is carried out in the presence of a suitable inorganic base such as hydroxides and carbonates of alkali metals, or in the presence of an organic base, particularly triethylamine or pyridine.

The most suitable reaction temperatures range from 0°C to room temperature, and the reaction time is between 5 minutes and 24 hours.

The resulting sulfonamide can be isolated by evaporating the solvent, adding water and eventually adjusting the pH so that it is obtained as a solid that can be isolated by filtration; or it can be extracted by a solvent immiscible in water such as chloroform and purified by chromatography or recrystallisation from a suitable solvent.

The compounds with the general formula (V) are commercially available or can be prepared according to standard methods or by methods analogous to those described in the literature [E.E. Gilbert, Synthesis, 1969, 1, 3] and the compounds with the general formula (VI) can be prepared according to standard methods or by methods analogous to those described in the literature [J.E. Macor, R. Post and K. Ryan, Synt Comm., 1993, 23, 1, 65-72.; J. Guillaume, C. Dumont, J. Laurent and N. Nédélec, Eur. J. Med. Chem., 1987, 22, 33-43; M.L. Saccarello, R. Stradi, Synthesis, 1979, 727].

Besides this process the invention further provides a further aspect in a process for the preparation of salts of compounds of general formula (I), wherein at least one compound of general formula (I) is reacted with an inorganic and/or organic acid, preferably in the presence of a suitable reaction medium. Suitable reaction media are the ones given above. Suitable inorganic acid are for example hydrochloric acid, hydrobromic acid, phosphoric acid, sulphuric acid, nitric acid. Suitable organic acids are e.g. citric acid, maleic acid, furmaric acid, tartaric acid or derivatives thereof, such as p-toluenesulfonic acid, methanesulfonic acid or camphersulfonic acid.

In yet a further aspect the present invention also provides a process for the preparation of salts of compounds of general formula (I), wherein at least one compound of general formula (I) having at least one acidic group is reacted with one or more suitable bases, preferably in the presence of suitable reaction medium. Suitable bases are e.g. hydroxides. Carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or linear C₁₋₄ alkyl radical.

Solvates, preferably hydrates, of the compounds of general formula (I), or corresponding stereoisomers, or corresponding salts may also be obtained by standard procedures known to those skilled in the art.

If the compounds of general formula (I) are obtained in form of a mixture of stereoisomers, particularly enantiomers or diastereomers, said mixtures may be separated by standard procedures known to those skilled in the art, e.g. chromatographic methods of crystallization with chiral reagents.

The purification and isolation of the compounds of general formula (I) or a corresponding stereoisomer, or a corresponding salt, or corresponding solvate respectively, if required may be carried out by conventional methods known to those skilled in the art, e.g. chromatographic methods or recrystallization.

The compounds of general formula (I), their stereoisomers or the respective salts or solvates are toxicologically acceptable and are therefore suitable as pharmaceutical active substances for the preparation of medicaments.

The present invention therefore also provides for a medicament comprising at least one compound of general formula (I) or (II), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and optionally one or more pharmaceutically acceptable adjuvants.

Furthermore, the present invention also provides for a pharmaceutical composition comprising at least one compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants, which is not yet formulated into a medicament.

Preferably the medicament is suitable for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

Preferably the medicament is also suitable for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition; especially
for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome.

The present invention also provides for the use of at least one compound of general formula (I), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

A highly preferred embodiment is also the use of an aryl-substituted sulfonamide compound according to the invention according to general formula II, 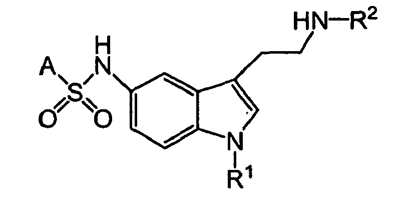 wherein
**A** represents
   a naphthyl, unsubstituted or substituted by 1 or 2 radicals selected from halogen, C₁-C₄ alkyl, CF₃, CHF₂, O-C₁-C₄ alkyl, OCF₃, OH, SH, or NH₂; and
**R¹** represents hydrogen or CH₃;
**R²** represents hydrogen, CH₃, C₂H₅, C₃H₇ or C₄H₉;
optionally in form of a salt, preferably a physiologically acceptable salt thereof, or a corresponding solvate, respectively;
for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition; especially
for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for
prophylaxis and/or treatment of gastrointestinal tract disorders, preferably of the irritable bowel syndrome, for prophylaxis and/or treatment of Metabolic Syndrome.

It is also preferred if the compound used according to formula II is a compound selected from
Naphthalene-1-sulfonic acid [3-(2-ethylamino-ethyl)-1H-indol-5-yl]-amide
Naphthalene-1-sulfonic acid [3-(2-methylamino-ethyl)-1 H-indol-5-yl]-amide
5-Chloro-naphthalene-2-sulfonic acid [3-(2-ethylamino-ethyl)-1 H-indol-5-yl]-amide
5-Chloro-naphthalene-2-sulfonic acid [3-(2-methylamino-ethyl)-1H-indol-5-yl]-amide
5-Chloro-naphthalene-2-sulfonic acid [1-methyl-3-(2-methylamino-ethyl)-1H-indol-5-yl]-amide
or from
Naphthalene-1-sulfonic acid [3-(2-amino-ethyl)-1H-indol-5-yl]-amide
Naphthalene-2-sulfonic acid [3-(2-amino-ethyl)-1H-indol-5-yl]-amide
5-Chloro-naphthalene-2-sulfonic acid [3-(2-amino-ethyl)-1H-indol-5-yl]-amide
5-Chloro-naphthalene-2-sulfonic acid [3-(2-amino-ethyl)-1-methyl-1H-indol-5-yl]-amide
optionally in form of a salt, preferably a physiologically acceptable salt thereof, or a corresponding solvate, respectively.

The medicament may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions of the present invention may also be administered topically or via a suppository.

The above mentioned compositions include preferably 1 to 60 % by weight of one or more of the compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, weight or degree of illness and so forth. The daily dosage for mammals including humans usally ranges from 1 milligram to 2000 milligram, preferably 1 to 1500 mg, more preferably 1 to 1000 mg of substance to be administered during one or several intakes.

The following examples are provided to illustrate the claimed invention and are not meant in any way to limit it

### Examples

The examples were prepared by the following general method:
A compound of general formula (V) or one of its suitably protected derivatives 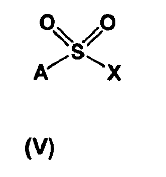
wherein A is as indicated above for the general formula (I) and X is an acceptable salient group including a halogen atom, particularly chlorine; is reacted with a 5-aminoindol with the general formula (VI), or one of its suitably protected derivatives; 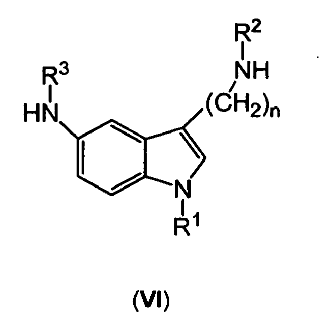 wherein n, R₁, R₂ and R₃ are as indicated above for the general formula (I); in order to obtain the corresponding sulfonamide and, optionally, eliminating from it the protective groups and / or forming a pharmacologically acceptable salt.

### Example 4: Preparation of 5-Chloro-naphthalene-2-sulfonic acid [3-(2-methylamino-ethyl)-1 H-indol-5-yl]-amide.

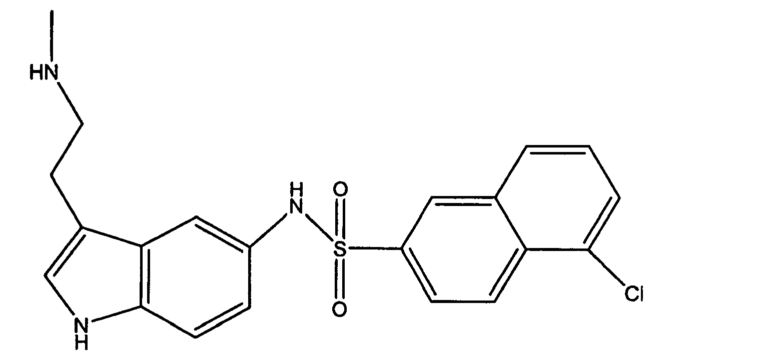

5-Chloro-naphthalene-2-sulfonic acid [3-(2-methylamino-ethyl)-1*H*-indol-5-yl]-amide

The synthesis was done according to the following reaction scheme: 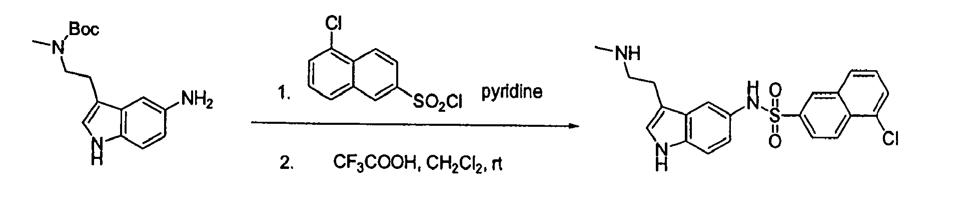 To a solution of 2.89 g (10 mMol) of tert-butyl 2-(5-amino-1H-indol-3-yl)ethyl(methyl)carbamate in 100 ml of pyridine is added dropwise at room temperature a solution of 10 mmol of 5-Chloro-naphthalene-2-sulfonyl chloride in 20 ml of pyridine. The reaction mixture is stirred at room temperature for 20 hours. It is then evaporated to dryness, slightly alkalinised with diluted ammonia and dissolved in ethyl acetate. The organic phase is washed with water and a saturated solution of sodium bicarbonate, it is separated and dried with anhydrous sodium sulphate. The organic solution is evaporated to dryness and the resulting Boc-protected sulfonamide is treated with a 5% solution of trifluoroacetic acid in dichloromethane, to yield 5-Chloro-naphthalene-2-sulfonic acid [3-(2-methylamino-ethyl)-1H-indol-5-yi]-amide.

Examples 1 to 3 and 5 to 9 are prepared in an analogue manner.

### Example 1:

### Naphthalene-1-sulfonic acid [3-(2-ethylamino-ethyl)-1H-indol-5-yl]-amide

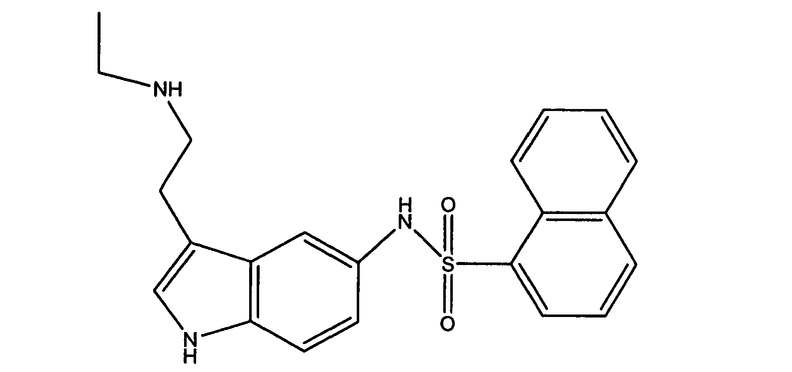

### Naphthalene-1-sulfonic acid [3-(2-ethylamino-ethyl)-1H-indol-5-yl]-amide

### Example 2:

### Naphthalene-1-sulfonic acid [3-(2-methylamino-ethyl)-1H-indol-5-yl]-amide

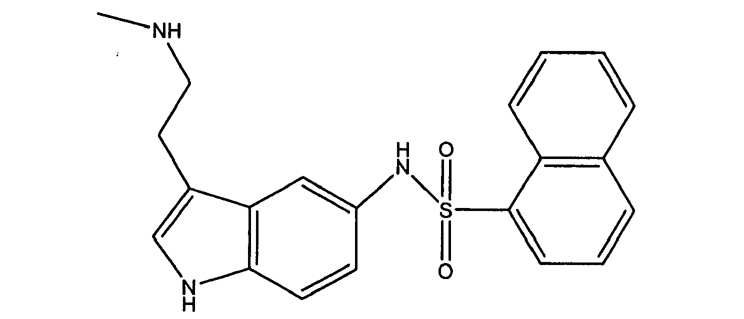

### Naphthalene-1-sulfonic acid [3-(2-methylamino-ethyl)-1H-indol-5-yl]-amide

### Example 3:

5-Chloro-naphthalene-2-sulfonic acid [3-(2-ethylamino-ethyl)-1H-indol-5-yl]-amide

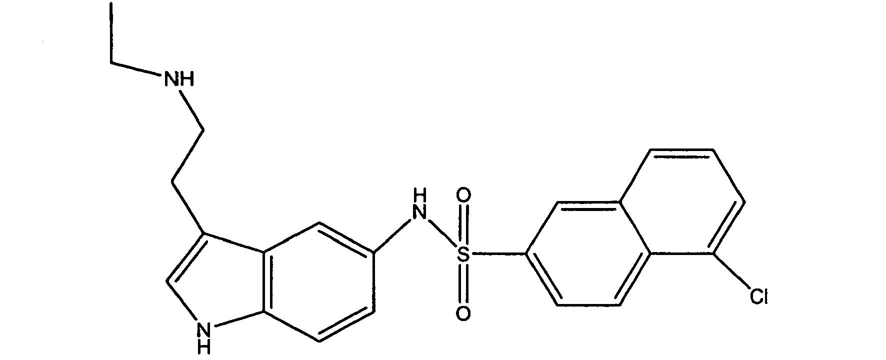

5-Chloro-naphthalene-2-sulfonic acid [3-(2-ethylamino-ethyl)-1*H*-indol-5-yl]-amide

### Example 5:

### Naphthalene-1-sulfonic acid [3-(2-amino-ethyl)-1H-indol-5-yl]-amide

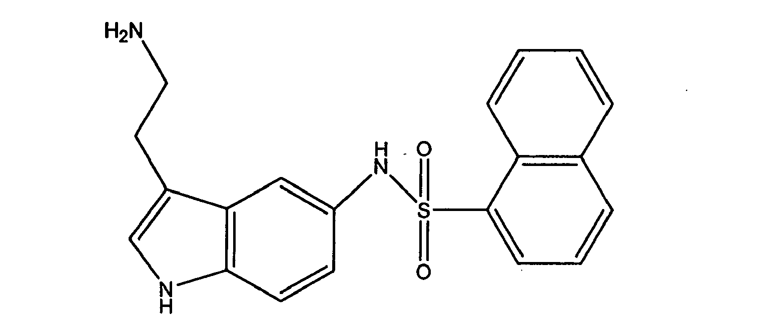

### Naphthalene-1-sulfonic acid [3-(2-amino-ethyl)-1H-indol-5-yl]-amide

### Example 6:

### 5-Chloro-naphthalene-2-sulfonic acid [1-methyl-3-(2-methylamino-ethyl)-1H-indol-5-yl]-amide

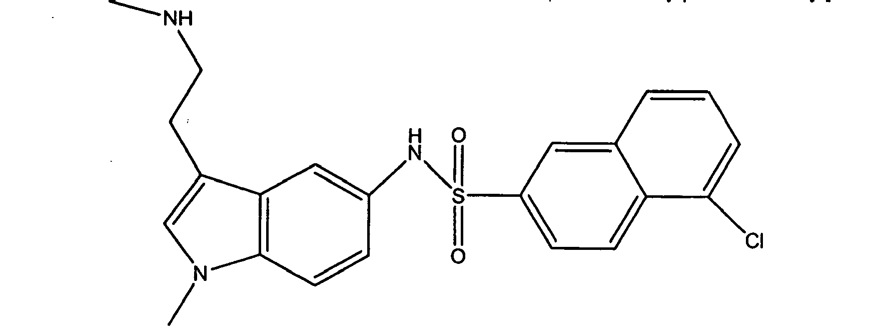

### 5-Chloro-naphthalene-2-sulfonic acid [1-methyl-3-(2-methylamino-ethyl)-1H-indol-5-yl]-amide

### Example 7:

### Naphthalene-2-sulfonic acid [3-(2-amino-ethyl)-1H-indol-5-yl]-amide

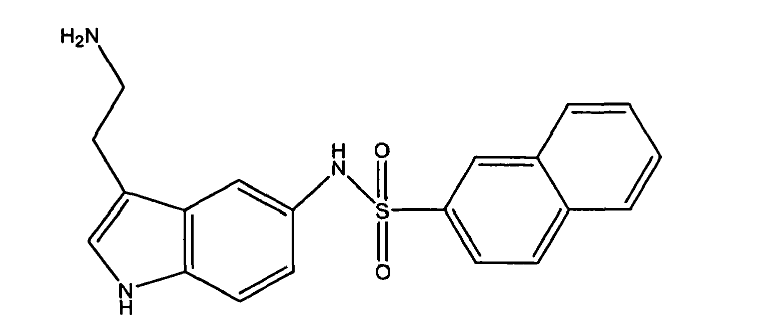

Naphthalene-2-sulfonic acid [3-(2-amino-ethyl)-1*H*-indol-5-yl]-amide

### Example 8:

### 5-Chloro-naphthalene-2-sulfonic acid [3-(2-amino-ethyl)-1 H-indol-5-yl]-amide

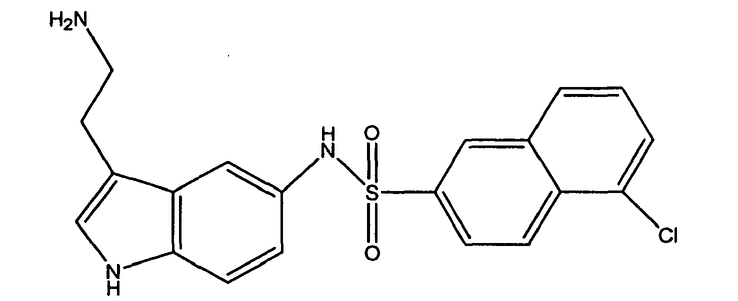

### 5-Chloro-naphthalene-2-sulfonic acid [3-(2-amino-ethyl)-1H-indol-5-yl]-amide

### Example 9:

### 5-Chloro-naphthalene-2-sulfonic acid [3-(2-amino-ethyl)-1-methyl-1H-indol-5-yl]-amide

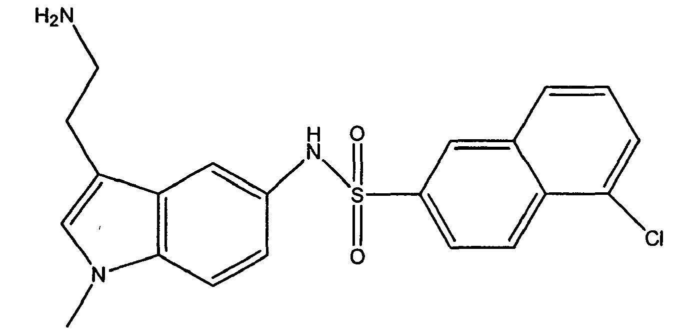

### 5-Chloro-naphthalene-2-sulfonic acid [3-(2-amino-ethyl)-1-methyl-1H-indol-5-yl]-amide

The examples are also listed in the following table:

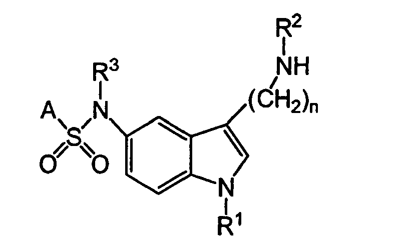

| Ex | R₁ | R₂ | n | R₃ | A | Salt | ¹H-NMR (300 MHz),δ (solvent) |
|---|---|---|---|---|---|---|---|
| 1 | H | C₂H₅ | 2 | H | 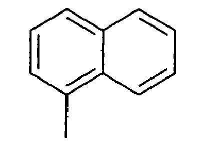 | | 0.96 (t, 3H, J = 7.2 Hz); 2.48-2.50 (m, 2H); 2.55 (bb, 4H); 6.63 (dd, 1 H, J = 8.7, 1.8 Hz); 7.01 (s, 1 H); 7.03 (m, 2H); 7.48 (t, 1H, J = 7.8 Hz); 7.66 (m, 2H); 8.02 (m, 2H); 8.11 (d, 1H, J = 8.1 Hz); 8.77 (d, 1H, J = 8.4 Hz); 10.67 (s, 1 H) (DMSO-d6) |
| 2 | H | CH₃ | 2 | H | 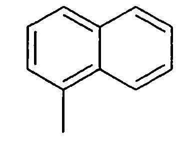 | | 2.23 (s, 3H); 2.48-2.56 (m, 4H); 6.64 (dd, 1H, J = 8.7, 1.8 Hz); 7.00 (s, 1 H); 7.03 (m, 2H); 7.49 (t, 1H, J = 7.8 Hz); 7.60-7.72 (m, 2H); 8.03 (m, 2H); 8.11 (d, 1H, J = 8.4 Hz; 8.78 (d, 1H, J = 8.4 Hz); 10.67 (s, 1 H) (DMSO-d6) |
| 3 | H | C₂H₅ | 2 | H | 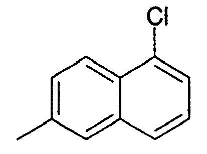 | | 0.92 (t, 3H, J = 6.9 Hz); 2.38-2.42 (m, 2H); 2.56-2.62 (m, 4H); 6.76 (d, 1H, J = 8.7 Hz); 7.05-7.14 (m, 3H); 7.58 (t, 1H, J = 7.8 Hz); 7.83 (d, 1H, J = 7.5 Hz); 7.89 (d, 1H, J = 8.7 Hz); 8.06 (d, 1H, J = 8.1 Hz); 8.28 (d, 1H, J = 9.0 Hz); 8.35 (s, 1 H); 10.75 (s, 1 H) (DMSO-d6) |

| | | | | | A | Salt | ¹H-NMR (300 MHz),δ (solvent) |
|---|---|---|---|---|---|---|---|
| 4 | H | CH₃ | 2 | H | 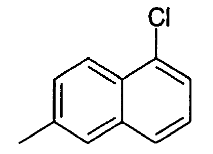 | | 2.16 (s, 3H); 2.50-2.63 (m, 4H); 6.76 (dd, 1 H, J = 8.7,1.8 Hz); 7.04 (d, 1 H, J = 2.4 Hz); 7.11 (s, 1 H); 7.12 (d, 1H, J = 5.7 Hz); 7.57 (t, 1 H, J = 4.8 Hz); 7.82 (dd, 1 H, J = 7.5, 0.9 Hz); 7.90 (dd, 1H, J = 9.0,1.8 Hz); 8.07 (d, 1H, J = 8.4 Hz); 8.28 (d, 1 H, J = 9.0 Hz); 8.36 (d, 1 H, J =1.8 Hz); 10.74 (s, 1H) (DMSO-d6) |
| 5 | H | H | 2 | H | 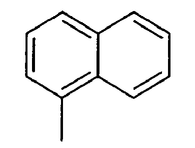 | | 2.79-2.88 (m, 4H); 6.57 (dd, 1H, J = 8.7,1.8 Hz); 7.04 (d, 1H, J = 8.7 Hz); 7.13-7.19 (m, 2H); 7.51 (t, 1H, J = 7.8 Hz); 7.64-7.71 (m, 2H); 8.03-8.08 (m, 2H); 8.14 (d, 1 H, J = 8.1 Hz); 8.77 (d, 1H, J = 8.4 Hz); 10.85 (s, 1 H) (DMSO-d6) |
| 6 | CH₃ | CH₃ | 2 | H | 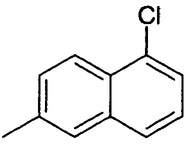 | | 2.36 (s, 3H); 2.77 (bb, 4H); 3.62 (s, 3H); 6.79 (dd, 1 H, J = 8.7, 2.1 Hz); 7.11 (s, 1 H); 7.20 (d, 1 H, J = 9.0 Hz); 7.25 (d, 1H, J =1.5 Hz); 7.60 (t, 1H, J = 7.5 Hz); 7.85 (dd, 1H, J = 7.5, 1.2 Hz); 7.91 (dd, 1H, J = 9.0, 1.8 Hz); 8.10 (d, 1H, J = 8.4 Hz); 8.31 (d, 1 H, J = 9.0 Hz); 8.39 (d, 1 H, J =1.8 Hz) (DMSO-d6) |
| 7 | H | H | 2 | H | 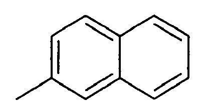 | | 2.58-2.64 (m, 4H); 6.74 (dd, 1H, J = 8.7, 2.1 Hz); 7.03 (d, 1 H, J = 2.1 Hz); 7.09 (d, 1 H, J = 8.7 Hz); 7.17 (d, 1 H, J = 1.5 Hz); 7.55-7.66 (m, 2H); 7.73 (dd, 1 H, J = 8.4, 1.5 Hz); 7.95 (d, 1 H, J = 7.8 Hz); 8.01-8.04 (m, 2H); 8.27 (s, 1 H); 10.71 (s, 1 H) (DMSO-d6) |
| 8 | H | H | 2 | H | 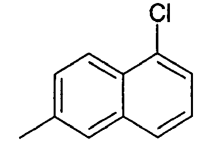 | | 2.66-2.74 (m, 4H); 6.71 (dd, 1H, J = 8.4, 2.1 Hz); 7.10 (m, 2H); 7.22 (d, 1 H, J = 1.8 Hz); 7.58 (t, 1H, J = 9.0 Hz); 7.83 (dd, 1H, J = 7.5, 1.2 Hz); 7.90 (dd, 1 H, J = 9.0, 1.8 Hz); 8.07 (d, 1H, J = 8.4 Hz); 8.28 (d, 1H, J = 9.0 Hz); 8.37 (d, 1 H, J = 1.8 Hz); 10.79 (s, 1 H) (DMSO-d6) |
| 9 | CH₃ | H | 2 | H | 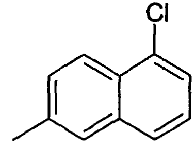 | | 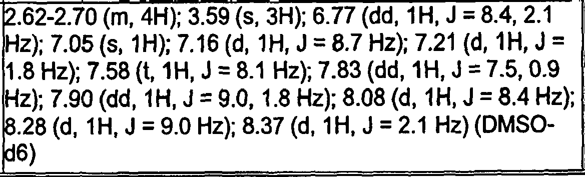 |

### BIOLOGICAL ASSAYS

### BINDING WITH SEROTONIN RECEPTOR 5HT₆

Cell membranes of HEK-293 cells expressing the 5HT₆ human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytriptamine-7 Receptors. The Journal of Pharmacology and Experimental Therapeutics, 1994, 268, 1403] with slight changes. The commercial membrane is diluted (1:40 dilution) with the binding buffer: 50 mM Tris-HCI, 10 mM MgCl₂, 0.5 mM EDTA (pH 7.4). The radioligand used is [³H]-LSD at a concentration of 2.7 nM with a final volume of 200 µl. incubation is initiated by adding 100 µl of membrane suspension, (≈ 22.9 µg membrane protein), and is prolonged for 60 minutes at a temperature of 37°C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Nonspecific binding is determined in the presence of 100 µM of serotonin. Tests were made in triplicate. The inhibition constants (Kₗ, nM) were calculated by non-linear regression analysis using the program EBDA/LIGAND [Munson and Rodbard, Analytical Biochemistry, 1980, 107, 220]. The following table shows results indicative of binding for some of the compounds object of the present invention.

| **Table** | | | |
|---|---|---|---|
| **Example** | **Binding % Inhibition 100 nM** | **Binding % Inhibition 10 nM** | **Binding 5HT₆ K, (nM)** |
| 1 | | | 3.0 |
| 2 | 91.2 | 83.0 | 2.9 |
| 3 | 82.9 | 60.1 | 2.0 |
| 4 | 73.5 | 64.9 | 2.1 ± 1.3 |
| 5 | 76.9 | 41.6 | 26.4 |
| 6 | 79.8 | 39.7 | |
| 7 | 84.6 | 66.0 | 7.1 ± 1.1 |
| 8 | 86.1 | 65.6 | 1.4 |
| 9 | 78.0 | 34.1 | |

The daily doses in human medicine are between 1 milligram and 500 milligrams of product, which can be given in one or more administrations. The compositions are prepared in forms compatible with the administration means used, such as sugar-coated pills, tablets, capsules, suppositories, solutions or suspensions. These compositions are prepared by known methods and comprise between 1 and 60% by weight of the active principle (compound with the general formula I) and 40 to 99% by weight of a suitable pharmaceutical vehicle compatible with the active principle and the physical form of the composition used. By way of example, the formula of a tablet containing a product of the invention is shown.

| Example of formula per tablet: | |
|---|---|
| Example 4 | 5 mg |
| Lactose | 60 mg |
| Crystalline cellulose | 25 mg |
| K 90 Povidone | 5 mg |
| Pregelatinised starch | 3 mg |
| Colloidal silicon dioxide | 1 mg |
| Magnesium stearate | 1 mg |
| Total weight per tablet | 100 mg |

## Claims

1. An aryl-substituted sulfonamide compound according to general formula (1) wherein
A represents an optionally at least mono substituted aryl radical, which may be bonded via an optionally at least mono-substituted or unsaturated alkylene group; an optionally at least monosubstituted alkyl-diaryl radical or an optionally at least monosubstituted group 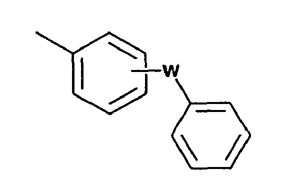 , wherein **W** represents a bond between the two rings, CH₂, O, S and NH;
**R¹** represents hydrogen, or C₁-C₄ alkyl or a benzyl radical;
**R²** represents hydrogen, or C₁-C₄ alkyl;
**R³** represents hydrogen or C₁-C₄ alkyl;
**n** represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
with the proviso that
• if R¹, R² and R³ are all H, A may only be alkyl- or halogen-substituted naphthyl or unsubstituted β-naphthyl, an optionally at least monosubstituted alkyl-diaryl radical or an optionally at least monosubstituted group

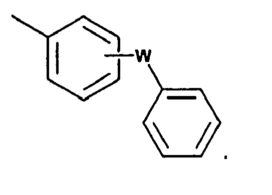

2. An aryl-substituted sulfonamide compound according to claim 1, wherein
**A** represents an optionally at least monosubstituted naphthyl or
a group selected from among: 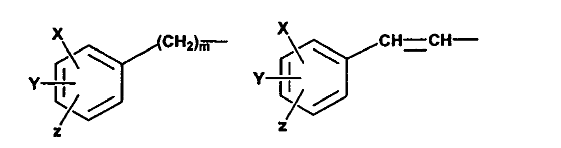 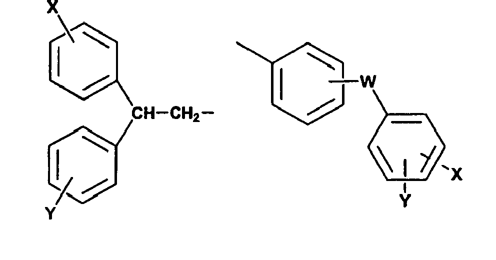 with
**X**, **Y** and **Z** independently representing hydrogen, fluorine, chlorine, bromine, lodine, a C₁-C₄ alkyl, a C₁-C₄,alkoxy, a C₁-C₄ alkylthio, trifluoromethyl, cyano, nitro, OH, SH and NH₂;
**W** representing a bond between the two rings, CH₂, O, S and NH;
**m** represents 0, 1, 2, 3 or 4.

3. An aryl-substituted sulfonamide compound according to any of claims 1 or 2 , wherein the compound is a compound according to formula I 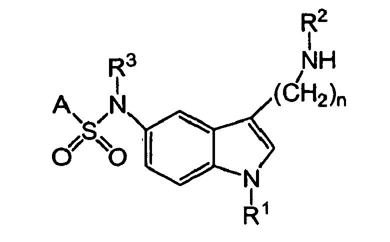 , wherein
**A** represents an optionally at least mono-substituted naphthyl;
**R¹** represents hydrogen, or C₁-C₄ alkyl;
**R²** represents hydrogen, or C₁-C₄ alkyl;
**R³** represents hydrogen or C₁-C₄ alkyl;
**n** represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio,
or a salt, preferably a physiologically acceptable salt thereof, or a corresponding solvate, respectively;
with the proviso that
if R¹, R² and R³ are all H, A may only be alkyl- or halogen-substituted naphthyl or unsubstituted β-naphthyl.

4. An aryl-substituted sulfonamide compound according to claim 3, wherein
**A** represents
a naphthyl, unsubstituted or substituted by 1 or 2 radicals selected from halogen, C₁-C₄ alkyl, O-C₁-C₄ alkyl, OCF₃, CF₃, CHF₂, CN, NO₂, OH, SH, NH₂, phenyl, optionally at least monosubstituted by halogen, C₁-C₄ alkyl, O-C₁-C₄ alkyl, OH, SH, NH₂, or a monocyclic heterocyclic ring system with 5 or 6 ring members containing 1 or 2 atoms of oxygen, nitrogen or sulphur as ring member, at least monosubstituted by halogen, C₁-C₄ alkyl, O-C₁-C₄ alkyl, OH, SH, NH₂ ;
preferably
a naphthyl, unsubstituted or substituted by 1 or 2 radicals selected from halogen, C₁-C₄ alkyl, CF₃, CHF₂, O-C₁-C₄ alkyl, OCF₃, OH, SH, or NH₂.

5. An aryl-substituted sulfonamide compound according to any of claims 1 or 2 or 3 to 4,
wherein
**R¹** represents hydrogen, CH₃, C₂H₅, C₃H₇ or C₄H₉; preferably hydrogen, methyl, ethyl, n-propyl, i-propyl, n-butyl, I-butyl and t-butyl; more preferably hydrogen, methyl or ethyl; most preferably hydrogen or methyl.

6. An aryl-substituted sulfonamide compound according to any of claims 1 or 2 or 3 to 5,
wherein
**R²** represents hydrogen, CH₃, C₂H₅, C₃H₇ or C₄H₉; preferably hydrogen, methyl, ethyl, n-propyl, i-propyl, n-butyl, i-butyl and t-butyl; more preferably hydrogen, methyl or ethyl.

7. An aryl-substituted sulfonamide compound according to any of claims 1 or 2 or 3 to 6,
wherein
**R³** represents hydrogen, CH₃, C₂H₅, C₃H₇ or C₄H₉; preferably hydrogen, methyl, ethyl, n-propyl, i-propyl, n-butyl, i-butyl and t-butyl; more preferably hydrogen, methyl or ethyl; most preferably hydrogen.

8. An aryl-substituted sulfonamide compound according to any of claims 1 or 2 or 3 to 7,
wherein
**n** represents 0, 1 or 2, preferably 2.

9. An aryl-substituted sulfonamide compound according to claim 1 or 3 according to general formula II, 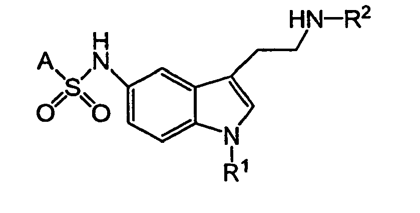 wherein
**A** represents
a naphthyl, unsubstituted or substituted by 1 or 2 radicals selected from halogen, C₁-C₄ alkyl, CF₃, CHF₂, O-C₁-C₄ alkyl, OCF₃, OH, SH, or NH₂; and
**R¹** represents hydrogen or CH₃;
**R²** represents hydrogen, CH₃, C₂H₅, C₃H₇ or C₄H₉;
optionally in form of a salt, preferably a physiologically acceptable salt thereof, or a corresponding solvate, respectively;
with the proviso that
if R¹, R² and R³ are all H, A may only be alkyl- or halogen-substituted naphthyl or unsubstituted β-naphthyl.

10. An aryl-substituted sulfonamide compound according to claim 9,
wherein
**R²** represents methyl, ethyl, n-propyl, i-propyl, n-butyl, i-butyl and t-butyl; preferably methyl or ethyl.
or
**R²** represents hydrogen.

11. An aryl-substituted sulfonamide compound according to claim 10, wherein the compound is selected from
Naphthalene-1-sulfonic acid [3-(2-ethylamino-ethyl)-1H-indol-5-yl]-amide
Naphthalene-1-sulfonic acid [3-(2-methylamino-ethyl)-1H-indol-5-yl]-amide
5-Chloro-naphthalene-2-sulfonic acid [3-(2-ethylamino-ethyl)-1H-indol-5-yl]-amide
5-Chloro-naphthalene-2-sulfonic acid [3-(2-methylamino-ethyl)-1H-indol-5-yl]-amide
5-Chloro-naphthalene-2-sulfonic acid [1-methyl-3-(2-methylamino-ethyl)-1H-indol-5-yl]-amide
or from
Naphthalene-2-sulfonic acid [3-(2-amino-ethyl)-1 H-indol-5-yl]-amide
5-Chloro-naphthalene-2-sulfonic acid [3-(2-amino-ethyl)-1 H-indol-5-yl]-amide
5-Chloro-naphthalene-2-sulfonic acid [3-(2-amino-ethyl)-1-methyl-1 H-indol-5-yl]-amide
optionally in form of a salt, preferably a physiologically acceptable salt thereof, or a corresponding solvate, respectively.

12. A medicament comprising an active substance according to one or more of claims 1 to 11 and optionally one or more pharmacologically acceptable adjuvants.

13. A medicament according to claim 12 for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

14. Use of a compound according to one or more of claims 1 to 11, for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

15. Use of an aryl-substituted sulfonamide compound according to general formula II, 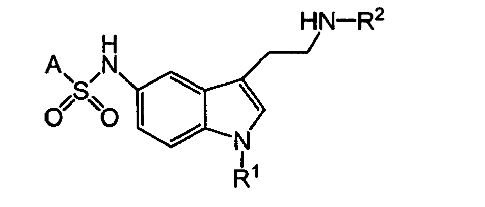 wherein
**A** represents
a naphthyl, unsubstituted or substituted by 1 or 2 radicals selected from halogen, C₁-C₄ alkyl, CF₃, CHF₂, O-C₁-C₄ alkyl, OCF₃, OH, SH, or NH₂; and
**R¹** represents hydrogen or CH₃;
**R²** represents hydrogen, CH₃, C₂H₅, C₃H₇ or C₄H₉;
optionally in form of a salt, preferably a physiologically acceptable salt thereof, or a corresponding solvate, respectively;
for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition; especially
for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome.

16. Use according to claim 15, **characterized in that** the aryl-substituted sulfonamide compound is selected from
Naphthalene-1-sulfonic acid [3-(2-ethytamino-ethyl)-1H-tndot-5-yl]-amide
Naphthalene-1-sulfonic acid [3-(2-methy)amino-ethyl)-1H-indol-5-yl]-amide
5-Chloro-naphthalene-2-sulfonic acid [3-(2-ethylamino-ethyl)-1H-indot-5-yl]-amide
5-Chloro-naphthalene-2-sulfonic acid [3-(2-methylamino-ethyl)-1H-indol-5-yl]-amide
5-Chloro-naphthalene-2-sulfonic acid [1-methyl-3-(2-methylamino-ethyl)-1H-indol-5-yl]-amide
or from
Naphthalene-1-sulfonic acid [3-(2-amino-ethyl)-1H-indol-5-yl]-amide
Naphthalene-2-sulfonic acid [3-(2-amino-ethyl)-1H-indol-5-yl]-amide
5-Chloro-naphthalene-2-sulfonic acid [3-(2-amino-ethyl)-1H-indol-5-yl]-amide
5-Chloro-naphthalene-2-sulfonic acid [3-(2-amino-ethyl)-1-methyl-1H-indol-5-yl]-amide
optionally in form of a salt, preferably a physiologically acceptable salt thereof, or a corresponding solvate, respectively.
